# EUROPÄISCHE PATENTANMELDUNG

(11) **EP 1 640 401 A2**
(43) Veröffentlichungstag der Anmeldung: **29.03.2006**
(21) Anmeldenummer: 05020413.0
(22) Anmeldetag: 20.09.2005
(51) Int. Cl.: C08G 65/28

(54) **Polyethylenglykol und Alkoholethoxylate und deren Herstellung**

(30) Priorität: 24.09.2004 DE 102004046355; 09.12.2004 DE 102004059222
(71) Anmelder: Clariant Produkte (Deutschland) GmbH, 65929 Frankfurt am Main (DE)
(72) Erfinder: Henning, Torsten, Dr., 19057 Schwerin (DE); Wagner, Rainer, DI, 84508 Burgkirchen (DE); Vybiral, Reinhard, Dr., 84508 Burgkirchen (DE); Berchthold, Peter, Dr., 84508 Burgkirchen (DE)

(57) **Zusammenfassung**

Es werden Polyethylenglykole und Alkoholethoxylate mit niedrigem Gehalt an Ethylenoxid von maximal 0,2 ppm sowie ein Verfahren zur Herstellung derartiger Substanzen beschrieben.

## Beschreibung

Die Erfindung betrifft neue Polyethylenglykole sowie Alkoholethoxylate mit einem besonders niedrigen Ethylenoxidgehalt und ein Verfahren zu deren Herstellung.

Polyethylenglykole der Formel H(OCH₂CH₂)ₙOH mit n gleich 4 bis 900 entsprechend mittleren Molmassen von 180 bis 40000 g/mol, die allgemein durch Polymerisation von Ethylenoxid an Wasser oder mehrwertigen Alkoholen hergestellt werden, werden aufgrund ihrer interessanten Eigenschaften in einer Vielzahl von Anwendungsgebieten eingesetzt. Bei einer großen Zahl dieser Anwendungen kommt das Polyethylenglykol in oberflächlichen Kontakt mit der Haut von Lebewesen, speziell Menschen, oder wird auch Menschen oder Tieren oral oder parenteral verabreicht. Solche Anwendungen sind beispielsweise Lösungsmittel für Wirkstoffe, Aromastoffe oder Riechstoffe in medizinischen Tropfen, Injektionslösungen, Nahrungsergänzungsmitteln, Tabletten, Salben, Sticks, Suppositorien oder Gelatinekapseln; Weichmacher für Überzüge von Filmtabletten; Bindemittel in Tabletten; Feuchthaltemittel in Zahnpasten; Feuchtigkeitsspender und/oder Konditioniermittel in Duschbädern, Shampoos, Cream-rinse-Spülungen, Treatment-Haarkuren, Seifen, Flüssigseifen, Haarsprays, Haargelen, After Shave-Produkten, Gesichtsmasken, Sonnenschutzprodukten, Cremes oder Lotionen; Bestandteil von mehrphasigen Produkten wie Zweiphasenduschbädern, Zweiphasenschaumbädern oder Dreiphasenölbädern; sowie Wirkstoff in Augentropfen, Laxantien oder antiapoptotisch wirkenden Lösungen.

Produkte mit ähnlichen Anwendungsgebieten erhält man allgemein durch Polymerisation von Ethylenoxid an Alkoholen.

Wichtig für diese Anwendungen ist es, den Gehalt an für den lebenden Organismus schädlichen Stoffen wie z.B. den Restmonomergehalt so gering wie irgend möglich zu halten. Im vorliegenden Fall der Polyethylenglykole oder Alkoholethoxylate ist das verwendete Monomer Ethylenoxid. Weiterhin gewünscht ist es, auch den Gehalt an Nebenprodukten so gering wie möglich zu halten. Im Fall der Herstellung von Polyethylenglykolen und Alkoholethoxylaten unter Verwendung von Ethylenoxid ist ein schädliches Nebenprodukt beispielsweise 1,4-Dioxan.

Für den Einsatz in pharmazeutischen Produkten fordert deshalb die Monografie 07/2003:1444 "Macrogole" in der Europäischen Pharmakopoe (Ph. Eur.) 4.5 (gültig seit Juli 2003) sowie die Monografie "Polyethylene glycols" in der United States Pharmacopoeia/National Formulary (USP/NF) 16/28 einen maximalen Ethylenoxidrestgehalt, bestimmt per Gaschromatographie, von maximal 1 ppm. Für Dioxan verlangen die oben genannten Monografien einen Grenzwert von maximal 10 ppm.

Marktübliches Polyethylenglykol und marktübliche Alkoholethoxylate zeigen auch tatsächlich diesen niedrigen Ethylenoxidrestgehalt mit Werten von ca. 0,8 bis 1,0 ppm sowie den geforderten niedrigen Dioxanrestgehalt mit Werten von ca. 1 bis 10 ppm.

In der Nahrungsmittelindustrie wird jedoch inzwischen gemäß EU Comission Directive 2003/95/EC vom 27. Oktober 2003 als Reinheitskriterium für Nahrungsmittelzusatzstoffe gemäß Scientific Commitee on Food Opinion vom 6. Mai 2002 ein strengerer Grenzwert von maximal 0,2 ppm Restethylenoxid in ethoxylierten Substanzen und damit eingeschlossen Polyethylenglykole, im einzelnen Polyethylenglykol 6000, gefordert. Solches Polyethylenglykol und entsprechende Alkoholethoxylate sind aber auf dem Markt bisher nicht bekannt. Weiterhin ist bisher kein Verfahren zur Herstellung von Polyethylenglykolen und Alkoholethoxylaten mit diesem niedrigen Restgehalt an Ethylenoxid von maximal 0,2 ppm bekannt.

Aufgabe der Erfindung war es daher, Polyethylenglykole der Formel H(OCH₂CH₂)ₙOH mit n gleich 4 bis 900 entsprechend mittleren Molmassen von 180 bis 40000 g/mol, die den oben genannten niedrigen Restgehalt an Ethylenoxid von maximal 0,2 ppm zeigen, zur Verfügung zu stellen.

Weitere Aufgabe der Erfindung war es auch, entsprechende Alkoholethoxylate, die ebenfalls den oben genannten niedrigen Restgehalt an Ethylenoxid von maximal 0,2 ppm zeigen, zur Verfügung zu stellen. Überraschenderweise wurde nun gefunden, dass diese Aufgaben gelöst werden, wenn ein Glykol oder ein Monoalkohol in Gegenwart eines Katalysators mit Ethylenoxid ethoxyliert und die erhaltene Reaktionsmischung anschließend mit Wasserdampf nachbehandelt und gegebenenfalls getrocknet oder einer Wasserwäsche und Trocknung unterzogen wird.

Gegenstand der Erfindung ist demnach ein Polyethylenglykol oder ein Alkoholethoxylat der Formel (I)

R(OCH₂CH₂)ₙOH (I)

worin
R H, einen linearen oder verzweigten Alkylrest mit 1 bis 30 Kohlenstoffatomen, einen linearen oder verzweigten Alkenylrest mit 2 bis 30 Kohlenstoffatomen oder einen Arylrest, der auch mit 1 bis 3 linearen oder verzweigten Alkylgruppen mit jeweils 1 bis 12 Kohlenstoffatomen substituiert sein kann, und
n 4 bis 900 entsprechend mittleren Molmassen der (OCH₂CH₂)ₙOH-Gruppierungen von 180 bis 40000 g/mol bedeutet,
dadurch gekennzeichnet, dass die Polymere der Formel (I) einen Gehalt an Ethylenoxid von maximal 0,2 ppm aufweisen.

In einer bevorzugten Ausführungsform der Erfindung ist R gleich H.

In einer weiteren bevorzugten Ausführungsform der Erfindung ist R ungleich H.

Sofern R eine Alkylgruppe ist, hat diese vorzugsweise 1 bis 22 und besonders bevorzugt 1 bis 18 Kohlenstoffatome.

Sofern R eine Alkenylgruppe ist, hat diese vorzugsweise 6 bis 22 und besonders bevorzugt 8 bis 18 Kohlenstoffatome.

Der gegebenenfalls substituierte Arylrest ist vorzugsweise ein Phenylrest oder ein substituierter Phenylrest.

Die Alkylgruppen der substituierten Arylreste haben vorzugsweise 3 bis 10 Kohlenstoffatome und sind insbesondere ausgewählt aus Butyl- und Nonylgruppen.

Sofern R ungleich H ist, ist dieser Rest vorzugsweise ausgewählt aus Alkylresten.

Vorzugsweise ist n in dem erfindungsgemäßen Polyethylenglykol oder Alkoholethoxylat der Formel (I) 32 bis 800 entsprechend einer mittleren Molmasse der (OCH₂CH₂)ₙOH-Gruppierungen von 1500 bis 35000 g/mol.

Der Gehalt an Ethylenoxid in dem erfindungsgemäßen Polyethylenglykol oder Alkoholethoxylat der Formel (I) beträgt vorzugsweise maximal 0,1 ppm.

Weiterer Gegenstand der Erfindung ist ein Verfahren zur Herstellung der Polymere der Formel (I).

Das erfindungsgemäße Verfahren zur Herstellung des Polyethylenglykols oder Alkoholethoxylats der Formel (I) zeichnet sich dadurch aus, dass ein Glykol oder ein Alkohol der Formel (II)

R^{a}OH (II)

worin
R^{a} einen linearen oder verzweigten Alkylrest mit 1 bis 30 Kohlenstoffatomen, einen linearen oder verzweigten Alkenylrest mit 2 bis 30 Kohlenstoffatomen, wobei die Alkyl- oder Aklenylreste auch ethoxyliert sein können und in diesem Fall 1 bis 30 -CH₂CH₂O-Gruppen enthalten, oder einen Arylrest, der auch mit 1 bis 3 linearen oder verzweigten Alkylgruppen mit jeweils 1 bis 12 Kohlenstoffatomen substituiert sein kann,
bedeutet,
in Gegenwart eines Katalysators mit Ethylenoxid ethoxyliert und die erhaltene Reaktionsmischung anschließend entweder mit Wasserdampf nachbehandelt und gegebenenfalls getrocknet oder einer Wasserwäsche und Trocknung unterzogen wird.

Im Vergleich zu dem Rest R der Formel (I) kann R^{a} aus Formel (II) nicht H bedeuten. Ansonsten entsprechen die bevorzugten Reste R^{a} den oben genannten bevorzugten Resten R. Zudem kann R^{a} auch ein ethoxylierter Alkyl- oder Alkenylrest sein. In diesem Fall enthalten diese Reste vorzugsweise 1 bis 10 und besonders bevorzugt 1 bis 3 -CH₂CH₂O-Gruppen.

Die Trocknung findet vorzugsweise derart statt, dass das Wasser, das u.a. durch die Wasserdampfbehandlung oder die Wasserwäsche in die Reaktionsmischung eingebracht worden ist, durch Destillation entfernt wird. Diese Destillation kann gegebenenfalls unter gegenüber Atmosphärendruck vermindertem Druck stattfinden.

Vorteilhafterweise kann mit dem erfindungsgemäßen Verfahren auch ein niedriger Dioxangehalt im Produkt erzielt werden. Der Gehalt an Dioxan in dem erfindungsgemäßen Polyethylenglykol oder Alkoholethoxylat der Formel (I) ist vorzugsweise maximal 1 ppm, besonders bevorzugt maximal 0,5 ppm und insbesondere bevorzugt maximal 0,1 ppm.

Die Herstellung von Polyethylenglykol oder Alkoholethoxylat durch Ethoxylierung von Glykolen oder Monoalkoholen ist dem Fachmann bekannt und kann beispielsweise wie in "1,2-Epoxide Polymers, Preparation" in: Encyclopedia of polymer science and engineering, John Wiley & Sons, Inc., revised edition 1986, Seite 242 f. beschrieben durchgeführt werden.

Bisher bekannte Verfahren zur Herstellung von Polyethylenglykol oder Alkoholethoxylat durch Ethoxylierung von Glykolen oder Monoalkoholen, die aber ohne den Schritt der Nachbehandlung mit Wasserdampf und gegebenenfalls anschließender Trocknung oder ohne den Schritt der Wasserwäsche mit anschließender Trocknung durchgeführt werden, sind nicht geeignet, um den niedrigen Gehalt an Ethylenoxid von maximal 0,2 ppm zu erreichen. Insbesondere sind sie nicht dazu geeignet, einen niedrigen Gehalt an Ethylenoxid von maximal 0,2 ppm und gleichzeitig einen niedrigen Gehalt an Dioxan von maximal 1 ppm zu erzielen.

Zur Erniedrigung des Ethylenoxidgehalts aus dem Reaktionsprodukt Polyethylenglykol wird z.B. in der EP 1 245 608 A1 vorgeschlagen, den Gehalt an nicht abreagiertem Restethylenoxid durch Anlegen eines Vakuums zu erniedrigen. Durch eine derartige Vorgehensweise kann das Ethylenoxid aus der viskosen Polyglykolmischung jedoch nur bis zu einem Wert von ca. 0,8 bis 1,0 ppm erniedrigt werden, aber nicht bis zur gewünschten Grenze von maximal 0,2 ppm.

In einer weiteren bevorzugten Ausführungsform wird das erfindungsgemäße Verfahren derart ausgeführt, dass das Edukt ein Glykol ist und ausgewählt ist aus der Gruppe bestehend aus Monoethylenglykol und höheren Ethylenglykolen mit einer mittleren Molmasse von bis zu 10000 g/mol. Besonders bevorzugt ist das Glykol ausgewählt aus Mono-, Di- oder Triethylenglykol.

In einer besonders bevorzugten Ausführungsform des erfindungsgemäßen Verfahrens wird das Mono-, Di- oder Triethylenglykol vor der Reaktion durch Destillation gereinigt. Das Mono-, Di- oder Triethylenglykol wird hierbei wiederum bevorzugt durch Destillation aus einer Glykolmischung, besonders bevorzugt aus einer Glykolmischung, die aus der Reaktion von Ethylenoxid mit Wasser hergestellt worden ist, erhalten.

In einer außerordentlich bevorzugten Ausführungsform des erfindungsgemäßen Verfahrens wird Monoethylenglykol, das durch Destillation bei einem Druck von 0 bis 40 hPa und einer Temperatur von 90 bis 200°C, bevorzugt bei 5 bis 20 hPa und 100 bis 150°C, besonders bevorzugt bei 10 hPa und 120°C, aus einer Glykolmischung erhalten wurde, als Edukt verwendet.

In einer weiteren außerordentlich bevorzugten Ausführungsform des erfindungsgemäßen Verfahrens wird Diethylenglykol, das durch Destillation bei einem Druck von 0 bis 40 hPa und einer Temperatur von 100 bis 220°C, bevorzugt bei 5 bis 20 hPa und 110 bis 180°C, besonders bevorzugt bei 10 hPa und 120°C, aus einer Glykolmischung erhalten wurde, als Edukt verwendet. In einer weiteren außerordentlich bevorzugten Ausführungsform des erfindungsgemäßen Verfahrens wird Triethylenglykol, das durch Destillation bei einem Druck von 0 bis 40 hPa und einer Temperatur von 140 bis 250°C, bevorzugt bei 5 bis 10 hPa und 140 bis 160°C, besonders bevorzugt bei 5 hPa und 140°C, aus einer Glykolmischung erhalten wurde, als Edukt verwendet.

In einer weiteren bevorzugten Ausführungsform des erfindungsgemäßen Verfahrens ist das Edukt ein Alkohol der Formel (II), welcher ausgewählt ist aus Methanol, Methylglykol, Methyldiglykol, Ethanol, Propanol, Butanol, Phenol, Nonylphenol, Tributylphenol, C₁₁-Alkohol wie C₁₁-Oxoalkohol, Laurylalkohol, Oleylalkohol, C₁₄- und C₁₅-Alkoholen wie C₁₄/C₁₅-Oxoalkohol, Cetylalkohol, Cetearylalkohol, Stearylalkohol, Isotridecylalkohol, C₁₀- bis C₁₂-Alkoholen wie C₁₀/C₁₂-Ziegleralkohol, C₁₂- bis C₁₅-Alkohol wie C₁₂/C₁₅-Oxoalkohol und deren Mischungen.

In einer weiteren bevorzugten Ausführungsform des erfindungsgemäßen Verfahrens wird der Katalysator in trockener Form oder als Lösung eingesetzt.

Der in dem erfindungsgemäßen Verfahren verwendete Katalysator ist vorzugsweise aus Alkali- oder Erdalkalimetallhydroxid, besonders bevorzugt aus Natriumhydroxid oder Kaliumhydroxid ausgewählt.

Besonders bevorzugt wird trockenes oder destilliertes Monoethylenglykol, Diethylenglykol oder Triethylenglykol oder Alkohol der Formel (II) zusammen mit trockenem Alkali- oder Erdalkalimetallhydroxid, bevorzugt Natriumhydroxid oder Kaliumhydroxid, in dem erfindungsgemäßen Verfahren verwendet.

In einer weiteren bevorzugten Ausführungsform des erfindungsgemäßen Verfahrens wird die Mischung aus Glykol oder Alkohol der Formel (II) und Katalysator oder aus Glykol oder Alkohol der Formel (II) und Katalysatorlösung vor der Ethoxylierung getrocknet. Die Trocknung findet vorzugsweise unter Vakuum statt.

In einer weiteren bevorzugten Ausführungsform des erfindungsgemäßen Verfahrens wird die Ethoxylierung unter Stickstoffatmosphäre bei einer Temperatur von 100 bis 160°C durchgeführt. In einer weiteren bevorzugten Ausführungsform des erfindungsgemäßen Verfahrens wird die Reaktionsmischung bzw. der Katalysator nach der Wasserdampfbehandlung und gegebenenfalls der Trocknung oder der Wasserwäsche mit anschließender Trocknung mit Säure neutralisiert. Vorzugsweise ist die Säure ausgewählt aus Milchsäure, Essigsäure und Isononansäure.

In einer bevorzugten Ausführungsform des erfindungsgemäßen Verfahrens wird die Reaktionsmischung mit Wasserdampf nachbehandelt.

In einer weiteren bevorzugten Ausführungsform des erfindungsgemäßen Verfahrens wird der Wasserdampfbehandlung eine Trocknung nachgeschaltet.

In einer weiteren bevorzugten Ausführungsform des erfindungsgemäßen Verfahrens wird bei der Behandlung der Reaktionsmischung mit Wasserdampf so vorgegangen, dass der Druck im Reaktor vor der Einleitung des Wasserdampfs auf 5 - 500 mbar, vorzugsweise auf 10 - 350 mbar, besonders bevorzugt auf 10 - 200 mbar und insbesondere bevorzugt auf 50 - 120 mbar eingestellt und die Behandlung der Reaktionsmischung mit Wasserdampf bei einer Temperatur von 100 bis 180°C, vorzugsweise 110 bis 150°C, für 1 bis 180 Minuten, vorzugsweise 10 bis 90 Minuten, durchgeführt wird.

In einer besonders bevorzugten Ausführungsform wird der Druck im Reaktor vor der Einleitung des Wasserdampfs auf 10 - 200 mbar eingestellt und die Behandlung der Reaktionsmischung mit Wasserdampf bei einer Temperatur von 100 bis 180°C für 1 bis 180 Minuten durchgeführt. Darunter bevorzugt ist wiederum eine Ausführungsform, in der der Druck im Reaktor vor der Einleitung des Wasserdampfs auf 50 - 120 mbar eingestellt und die Behandlung der Reaktionsmischung mit Wasserdampf bei einer Temperatur von 110 bis 150°C für 10 bis 90 Minuten durchgeführt wird.

Durch die Einleitung des Wasserdampfs wird das zunächst angelegte Vakuum verschlechtert. Das Vakuum kann durch die Einleitung des Wasserdampfs je nach den gewählten Bedingungen z.B. um 50 - 300 mbar oder um 100 - 200 mbar verschlechtert werden.

In einer weiteren bevorzugten Ausführungsform des erfindungsgemäßen Verfahrens wird bei der Wasserwäsche so vorgegangen, dass die Temperatur auf 20 bis 90°C, vorzugsweise 25 bis 80 °C, abgekühlt und dann Wasser in Mengen von 1 bis 95 Gew.-%, vorzugsweise 5 bis 50 Gew.-%, bezogen auf die Reaktionsmischung, zugefügt wird. Die Wasserwäsche wird vorzugsweise unter Atmosphärendruck durchgeführt.

Weiterer Gegenstand der Erfindung ist auch ein Polyethylenglykol oder ein Alkoholethoxylat der Formel (I) erhältlich nach dem erfindungsgemäßen Verfahren.

Die nachfolgenden Beispiele dienen der näheren Erläuterung der Erfindung ohne sie jedoch darauf einzuschränken. Alle Prozentangaben sind Gewichtsprozente.

### Beispiel 1a: Herstellung von Polyethylenglykol mit mittlerer Molmasse von 200 g/mol

7491 kg Triethylenglykol, das durch schonende Destillation bei 5 hPa und 140°C gewonnen wurde, wurden mit 5 kg 50 %iger, wässriger Natriumhydroxidlösung als Katalysator versetzt und für 1 Stunde bei 110°C und Vakuum getrocknet. Dann wurden 2498 kg Ethylenoxid, gasförmig in Stickstoffatmosphäre zugegeben. Nach erfolgter Reaktion wurde der Druck auf 100 mbar eingestellt und anschließend Wasserdampf für 60 Minuten bei 130°C durch die Reaktionsmischung geleitet. Danach wurde die Reaktionsmischung mit 6 kg 90%iger Milchsäure neutralisiert. Der Ethylenoxidrestgehalt wurde mit 0,1 ppm und der Dioxanrestgehalt mit 0,1 ppm per Gaschromatographie bestimmt.

### Beispiel 1b: Herstellung von Polyethylenglykol mit mittlerer Molmasse von 200 g/mol

7491 kg Triethylenglykol, das durch schonende Destillation bei 5 hPa und 140°C gewonnen wurde, wurden mit 5 kg 50 %iger, wässriger Natriumhydroxidlösung als Katalysator versetzt und für 1 Stunde bei 110°C und Vakuum getrocknet. Dann wurden 2498 kg Ethylenoxid, gasförmig in Stickstoffatmosphäre zugegeben. Nach erfolgter Reaktion wurden 50 % Wasser bezogen auf die Reaktionsmischung bei 80 °C und Atmosphärendruck zugegeben. Nach erfolgter Trocknung durch Abdestillieren des Wassers wurde die Reaktionsmischung mit 6 kg 90%iger Milchsäure neutralisiert. Der Ethylenoxidrestgehalt wurde mit 0,1 ppm und der Dioxanrestgehalt mit 0,1 ppm per Gaschromatographie bestimmt.

### Vergleichsbeispiel 1: Herstellung von Polyethylenglykol mit mittlerer Molmasse von 200 g/mol

7491 kg Triethylenglykol, das durch schonende Destillation bei 5 hPa und 140°C gewonnen wurde, wurden mit 5 kg 50 %iger, wässriger Natriumhydroxidlösung als Katalysator versetzt und für 1 Stunde bei 110°C und Vakuum getrocknet. Dann wurden 2498 kg Ethylenoxid, gasförmig in Stickstoffatmosphäre zugegeben. Nach erfolgter Reaktion wurde bei 130°C ein Vakuum von 100 mbar für 60 Minuten angelegt. Danach wurde die Reaktionsmischung mit 6 kg 90%iger Milchsäure neutralisiert.
Der Ethylenoxidrestgehalt wurde mit 1,0 ppm und der Dioxanrestgehalt mit 10 ppm per Gaschromatographie bestimmt.

### Beispiel 2: Herstellung von Polyethylenglykol mit mittlerer Molmasse von 3000 g/mol

Zu 11380 kg eines mit NaOH alkalisch gestellten PEG 1500 (Polyethylenglykol mit mittlerer Molmasse von 1500 g/mol) wurden 13460 kg Ethylenoxid, gasförmig in Stickstoffatmosphäre zugegeben. Nach erfolgter Reaktion wurde der Druck auf 100 mbar eingestellt und anschließend Wasserdampf für 60 Minuten bei 130°C durch die Reaktionsmischung geleitet. Danach wurde die Reaktionsmischung mit 6 kg 90%iger Milchsäure neutralisiert.

Der Ethylenoxidrestgehalt wurde mit 0,1 ppm und der Dioxanrestgehalt mit 0,1 ppm per Gaschromatographie bestimmt.

### Vergleichsbeispiel 2: Herstellung von Polyethylenglykol mit mittlerer Molmasse von 3000 g/mol

Zu 11380 kg eines mit NaOH alkalisch gestellten PEG 1500 wurden 126720 kg Ethylenoxid, gasförmig in Stickstoffatmosphäre zugegeben. Nach erfolgter Reaktion wurde bei 130°C ein Vakuum von 100 mbar für 60 Minuten angelegt. Danach wurde die Reaktionsmischung mit 6 kg 90%iger Milchsäure neutralisiert.
Der Ethylenoxidrestgehalt wurde mit 1,0 ppm sowie der Dioxanrestgehalt mit 10 ppm per Gaschromatographie bestimmt.

## Patentansprüche

1. Substanz der Formel (I)
R(OCH₂CH₂)ₙOH (I)
worin
R H, einen linearen oder verzweigten Alkylrest mit 1 bis 30 Kohlenstoffatomen, einen linearen oder verzweigten Alkenylrest mit 2 bis 30 Kohlenstoffatomen oder einen Arylrest, der auch mit 1 bis 3 linearen oder verzweigten Alkylgruppen mit jeweils 1 bis 12 Kohlenstoffatomen substituiert sein kann, und
n 4 bis 900
bedeutet,
**dadurch gekennzeichnet, dass** sie einen Gehalt an Ethylenoxid von maximal 0,2 ppm aufweist.

2. Substanz nach Anspruch 1, **dadurch gekennzeichnet, dass** n 32 bis 800 ist.

3. Substanz nach Anspruch 1 oder 2, **dadurch gekennzeichnet, dass** der Gehalt an Ethylenoxid maximal 0,1 ppm beträgt.

4. Substanz nach einem oder mehreren der Ansprüche 1 bis 3 mit einem Gehalt an Dioxan von maximal 1 ppm.

5. Substanz nach Anspruch 4, **dadurch gekennzeichnet, dass** der Gehalt an Dioxan maximal 0,5 ppm beträgt.

6. Verfahren zur Herstellung einer Substanz nach einem oder mehreren der Ansprüche 1 bis 5, **dadurch gekennzeichnet, dass** ein Glykol oder ein Alkohol der Formel (II)
R^{a}OH (II)
worin
R^{a} einen linearen oder verzweigten Alkylrest mit 1 bis 30 Kohlenstoffatomen, einen linearen oder verzweigten Alkenylrest mit 2 bis 30 Kohlenstoffatomen, wobei die Alkyl- oder Aklenylreste auch ethoxyliert sein können und in diesem Fall 1 bis 30 -CH₂CH₂O-Gruppen enthalten, oder einen Arylrest, der auch mit 1 bis 3 linearen oder verzweigten Alkylgruppen mit jeweils 1 bis 12 Kohlenstoffatomen substituiert sein kann,
bedeutet,
in Gegenwart eines Katalysators mit Ethylenoxid ethoxyliert und die erhaltene Reaktionsmischung anschließend entweder mit Wasserdampf nachbehandelt und gegebenenfalls getrocknet oder einer Wasserwäsche und Trocknung unterzogen wird.

7. Verfahren nach Anspruch 6, **dadurch gekennzeichnet, dass** das Edukt ein Glykol ist und ausgewählt ist aus der Gruppe bestehend aus Monoethylenglykol und höheren Ethylenglykolen mit einer mittleren Molmasse von bis zu 10000 g/mol.

8. Verfahren nach Anspruch 6 oder 7, **dadurch gekennzeichnet, dass** das Edukt ein Glykol ist und ausgewählt ist aus Mono-, Di- oder Triethylenglykol.

9. Verfahren nach Anspruch 8, **dadurch gekennzeichnet, dass** das Mono-, Di- oder Triethylenglykol vor der Reaktion durch Destillation gereinigt worden ist.

10. Verfahren nach Anspruch 8 oder 9, **dadurch gekennzeichnet, dass** das Mono-, Di- oder Triethylenglykol durch Destillation aus einer Glykolmischung erhalten worden ist.

11. Verfahren nach Anspruch 10, **dadurch gekennzeichnet, dass** die Glykolmischung aus der Reaktion von Ethylenoxid und Wasser hergestellt worden ist.

12. Verfahren nach einem oder mehreren der Ansprüche 6 bis 11, **dadurch gekennzeichnet, dass** es sich bei dem Edukt um Monoethylenglykol handelt, welches durch Destillation bei einem Druck von 0 bis 40 hPa und einer Temperatur von 90 bis 200°C erhalten worden ist.

13. Verfahren nach einem oder mehreren der Ansprüche 6 bis 11, **dadurch gekennzeichnet, dass** es sich bei dem Edukt um Diethylenglykol handelt, welches durch Destillation bei einem Druck von 0 bis 40 hPa und einer Temperatur von 100 bis 220°C erhalten worden ist.

14. Verfahren nach einem oder mehreren der Ansprüche 6 bis 11, **dadurch gekennzeichnet, dass** es sich bei dem Edukt um Triethylenglykol handelt, welches durch Destillation bei einem Druck von 0 bis 40 hPa und einer Temperatur von 140 bis 250°C erhalten worden ist.

15. Verfahren nach Anspruch 6, **dadurch gekennzeichnet, dass** das Edukt ein Alkohol der Formel (II) ist, welcher ausgewählt ist aus Methanol, Methylglykol, Methyldiglykol, Ethanol, Propanol, Butanol, Phenol, Nonylphenol, Tributylphenol, C₁₁-Alkohol wie C₁₁-Oxoalkohol, Laurylalkohol, Oleylalkohol, C₁₄- und C₁₅-Alkoholen wie C₁₄/C₁₅-Oxoalkohol, Cetylalkohol, Cetearylalkohol, Stearylalkohol, Isotridecylalkohol, C₁₀- bis C₁₂-Alkoholen wie C₁₀/C₁₂-Ziegleralkohol, C₁₂- bis C₁₅-Alkohol wie C₁₂/C₁₅-Oxoalkohol und deren Mischungen.

16. Verfahren nach einem oder mehreren der Ansprüche 6 bis 15, **dadurch gekennzeichnet, dass** der Katalysator in trockener Form oder als Lösung eingesetzt wird.

17. Verfahren nach einem oder mehreren der Ansprüche 6 bis 16, **dadurch gekennzeichnet, dass** der Katalysator aus Alkali- oder Erdalkalimetallhydroxid ausgewählt ist.

18. Verfahren nach Anspruch 17, **dadurch gekennzeichnet, dass** der Katalysator aus Natriumhydroxid oder Kaliumhydroxid ausgewählt ist.

19. Verfahren nach einem oder mehreren der Ansprüche 6 bis 18, **dadurch gekennzeichnet, dass** die Mischung aus Glykol oder Alkohol der Formel (II) und Katalysator oder aus Glykol oder Alkohol der Formel (II) und Katalysatorlösung vor der Ethoxylierung getrocknet wird.

20. Verfahren nach einem oder mehreren der Ansprüche 6 bis 19, **dadurch gekennzeichnet, dass** die Ethoxylierung unter Stickstoffatmosphäre bei einer Temperatur von 100 bis 160°C durchgeführt wird.

21. Verfahren nach einem oder mehreren der Ansprüche 6 bis 20, **dadurch gekennzeichnet, dass** die Reaktionsmischung nach der Wasserdampfbehandlung und gegebenenfalls der Trocknung oder der Wasserwäsche mit anschließender Trocknung mit Säure neutralisiert wird.

22. Verfahren nach Anspruch 21, **dadurch gekennzeichnet, dass** die Säure ausgewählt ist aus Milchsäure, Essigsäure und Isononansäure.

23. Verfahren nach einem oder mehreren der Ansprüche 6 bis 22, **dadurch gekennzeichnet, dass** die Reaktionsmischung mit Wasserdampf nachbehandelt wird.

24. Verfahren nach Anspruch 23, **dadurch gekennzeichnet, dass** der Wasserdampfbehandlung eine Trocknung nachgeschaltet wird.

25. Verfahren nach Anspruch 23 oder 24, **dadurch gekennzeichnet, dass** die Wasserdampfbehandlung derart durchgeführt wird, dass der Druck vor der Einleitung des Wasserdampfs auf 5 - 500 mbar eingestellt und die Behandlung der Reaktionsmischung mit Wasserdampf bei einer Temperatur von 100 bis 180°C für 1 bis 180 Minuten durchgeführt wird.

26. Verfahren nach Anspruch 25, **dadurch gekennzeichnet, dass** die Behandlung der Reaktionsmischung mit Wasserdampf bei einer Temperatur von 110 bis 150°C für 10 bis 90 Minuten durchgeführt wird.

27. Substanz erhältlich nach einem Verfahren gemäß einem oder mehreren der Ansprüche 6 bis 26.
